# FASCICULE DE BREVET EUROPEEN

(11) **EP 3 643 342 B1**
(45) Date de publication et mention de la délivrance du brevet: **15.12.2021**
(21) Numéro de dépôt: 19205183.7
(22) Date de dépôt: 24.10.2019
(51) Int. Cl.: A61M 1/36, A61M 39/22

(54) **DISPOSITIF D'INTERFACE POUR LA RÉALISATION D'UNE HÉMODIALYSE**
SCHNITTSTELLENVORRICHTUNG FÜR DIE DURCHFÜHRUNG EINER HÄMODIALYSE
INTERFACE DEVICE FOR CARRYING OUT HAEMODIALYSIS

(30) Priorité: 26.10.2018 FR 1859965
(43) Date de publication de la demande: 29.04.2020
(73) Titulaire: Ubiplug, 14280 Saint-Contest (FR)
(72) Inventeur: JEAN, Eric, 14112 BIEVILLE-BEUVILLE (FR); MISSAIRE, Fabrice, 1428 LILLOIS (BE); THUAUDET, Sylvain, 14480 FRESNE-CAMILLY (FR)
(74) Mandataire: Lavialle, Bruno François Stéphane

(56) Documents cités:
- WO-A1-99/64088
- WO-A1-2009/001152
- US-A1- 2005 145 549
- US-A1- 2013 303 986

## Description

L'invention concerne le domaine de l'hémodialyse et en particulier un dispositif d'interface entre une machine à hémodialyse et des tubes destinés à être reliés au système circulatoire d'un patient.

### ARRIERE PLAN DE L'INVENTION

L'hémodialyse consiste en la circulation du sang d'un patient vers une machine à hémodialyse, puis après avoir traité le sang à l'aide de la machine à hémodialyse, le sang est restitué vers système circulatoire du patient.

La liaison entre la machine à hémodialyse et le patient se fait via au moins un tube et via un dispositif d'interface qui sert de connecteur amovible entre la machine à hémodialyse et le tube. Ce tube présente une extrémité qui débouche au niveau du système circulatoire.

Pour mettre en œuvre cette liaison, le personnel médical relie un premier conduit à la machine à hémodialyse et fait circuler un liquide dans ce premier conduit pour en chasser les gaz.

En parallèle, le personnel médical implante manuellement un cathéter doté dudit tube pour le relier au système circulatoire du patient puis il aspire du sang du patient via ce tube pour le remplir de sang et en évacuer les gaz.

Une fois le conduit et le cathéter purgés de leurs gaz, le conduit et le cathéter sont alors reliés mécaniquement entre eux via un dispositif d'interface. L'aspiration de sang vers la machine à hémodialyse peut alors commencer.

En fin de séance d'hémodialyse, le cathéter relié au patient est débranché et du sérum physiologique y est injecté pour restituer le sang se trouvant dans ce cathéter vers le système circulatoire du patient.

Toutes ces opérations sont reproduites à chaque fois que l'on veut relier le patient à la machine à hémodialyse via un cathéter. Ainsi, dans les cas où l'on implante un cathéter veineux pour admettre du sang vers le patient et un cathéter artériel pour prélever du sang du patient, on doit multiplier les opérations manuelles. Le risque de contamination du système circulatoire du patient s'en trouve augmenté.

Un dispositif d'interface pour réaliser une hémodialyse est présenté sur le document brevet US5713850A. Ce dispositif permet d'échanger du fluide avec le patient via un seul tube relié au patient. D'autres dispositifs d'interface de l'art antérieur sont par exemple divulgués dans les documents US2005/145549 A1, WO 99/64088 A1, WO2009/001152 A1 et US2013/303986 A1.

Il serait par conséquent utile de développer un dispositif d'interface minimisant le risque de contamination du système circulatoire du patient.

### OBJET DE L'INVENTION

Un objet de la présente invention est de fournir un dispositif d'interface entre une machine à hémodialyse et au moins un tube résolvant tout ou partie des inconvénients précités de l'art antérieur.

### RESUME DE L'INVENTION

A cette fin il est proposé selon l'invention un dispositif d'interface entre une machine à hémodialyse et au moins un tube veineux destiné à être relié au système circulatoire d'un patient pour transférer du sang de la machine à hémodialyse vers le système circulatoire comportant les caractéristiques techniques de la revendication indépendante 1. Ce dispositif d'interface comprend
- un premier port adapté à être relié à un port de sortie de la machine à hémodialyse;
- un port veineux pour injecter du sang vers le tube veineux ;
le dispositif d'interface étant adapté à sélectivement adopter une première configuration dans laquelle le passage de sang entre le premier port et le port veineux est interdit et une deuxième configuration dans laquelle le premier port est relié au port veineux pour permettre le passage de sang du premier port vers le port veineux.

Le dispositif d'interface selon l'invention est également adapté à former une interface entre la machine à hémodialyse et un tube artériel destiné à être relié audit système circulatoire du patient pour transférer du sang du système circulatoire vers la machine à hémodialyse, ce dispositif d'interface comprenant également :
- un deuxième port adapté à être relié à un port d'entrée de la machine à hémodialyse ;
- un port artériel pour recevoir du sang du patient provenant du tube artériel, le dispositif d'interface étant en outre adapté pour interdire le passage de sang entre le deuxième port et le port artériel lorsque le dispositif se trouve dans sa première configuration et pour autoriser le passage de sang entre le deuxième port et le port artériel lorsque le dispositif se trouve dans sa deuxième configuration.

Le dispositif d'interface selon l'invention comporte également un troisième port, le dispositif d'interface étant adapté pour sélectivement adopter une configuration de déverrouillage distincte desdites première et deuxième configurations, dans cette configuration de déverrouillage le troisième port étant relié à un seul desdits port veineux ou port artériel alors que le premier port et le deuxième port sont respectivement isolés vis-à-vis du port veineux et du port artériel, ces ports veineux et artériel étant également isolés l'un de l'autre.

Le dispositif d'interface permet de relier des tubes veineux et artériel à une même machine à hémodialyse tout en permettant à l'aide de ce seul dispositif d'interface de :
- soit simultanément interdire le passage de fluide entre la machine à hémodialyse et le tube veineux et entre la machine à hémodialyse et le tube artériel en plaçant le dispositif d'interface dans sa première configuration ;
- soit simultanément autoriser le passage de fluide entre la machine à hémodialyse et le tube veineux et entre la machine à hémodialyse et le tube artériel en plaçant le dispositif d'interface dans sa deuxième configuration.

On peut ainsi, avec un même dispositif d'interface, sélectivement autoriser ou interdire une liaison fluidique veineuse et une liaison fluidique artérielle.

Ceci facilite grandement les manipulations pour le personnel médical tout en réduisant le risque d'erreurs de manipulation.

Pour la compréhension de la présente invention, sauf indication contraire tout port du dispositif d'interface qui n'est pas explicitement mentionné comme relié à un autre port du dispositif d'interface doit être considéré comme isolé vis-à-vis de tous les autres ports du dispositif d'interface.

Par ailleurs, lorsque l'on indique que des ports donnés sont reliés entre eux, cela signifie qu'il y a une communication fluidique entre ces ports donnés.

De même, lorsque l'on indique que deux ports donnés sont isolés l'un de l'autre, cela signifie qu'il n'existe pas de communication fluidique entre ces ports donnés.

Le fluide transitant via le dispositif d'interface est un liquide. Par exemple, ce fluide est du sang, un dialysat, du sérum physiologique, un médicament sous forme liquide.

Selon un autre aspect, l'invention concerne un système d'hémodialyse comprenant une machine à hémodialyse et un dispositif d'interface selon l'un quelconque des modes de réalisations du dispositif d'interface décrit ci-après.

Dans ce système d'hémodialyse, le premier port Pm1 du dispositif d'interface est relié de manière amovible au port de sortie de la machine à hémodialyse et le deuxième port du dispositif d'interface est relié de manière amovible au port d'entrée de la machine à hémodialyse. La machine à hémodialyse comporte une pompe agencée pour faire circuler des fluides de son port d'entrée vers son port de sortie et le système d'hémodialyse comprend également un tube veineux et un tube artériel. Le tube veineux est relié au port veineux du dispositif d'interface. Ce tube veineux est destiné à être relié au système circulatoire d'un patient pour transférer, via le dispositif d'interface, du sang de la machine à hémodialyse vers le système circulatoire. Le tube artériel est relié au port artériel du dispositif d'interface. Ce tube artériel est destiné à être relié audit système circulatoire du patient pour transférer, via le dispositif d'interface, du sang du système circulatoire vers la machine à hémodialyse.

Le système d'hémodialyse selon l'invention est avantageux au moins pour les raisons énoncées ci-avant en référence au dispositif d'interface selon l'invention.

### BREVE DESCRIPTION DES DESSINS

D'autres caractéristiques et avantages de l'invention ressortiront clairement de la description qui en est faite ci-après, à titre indicatif et nullement limitatif, en référence aux dessins annexés, dans lesquels:
- La figure la présente un système d'hémodialyse 0 selon l'invention comprenant une machine à hémodialyse 2, un dispositif d'interface 1 selon l'invention et des tubes veineux et artériel X1, X2, ce dispositif d'interface 1 est ici contenu dans une seule enceinte E et est attaché aux tubes veineux et artériel X1, X2 et aux ports d'entrée et de sortie M1, M2 de la machine pour sélectivement autoriser ou interdire des liaisons fluidiques entre le port de la machine M1 et le tube X1 et entre le port M2 et le tube X2 ;

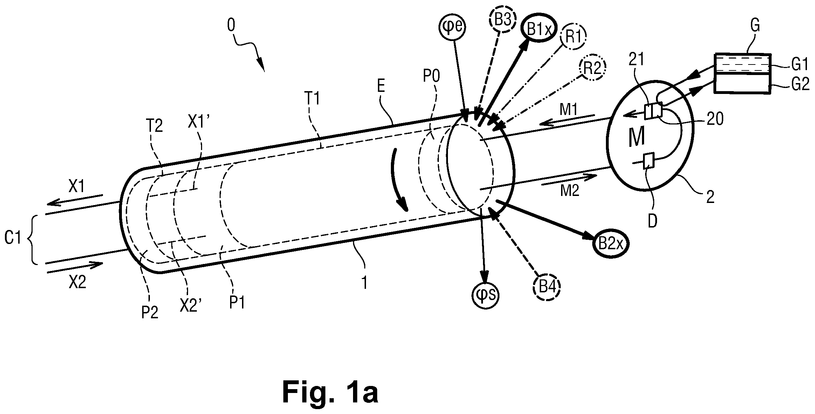

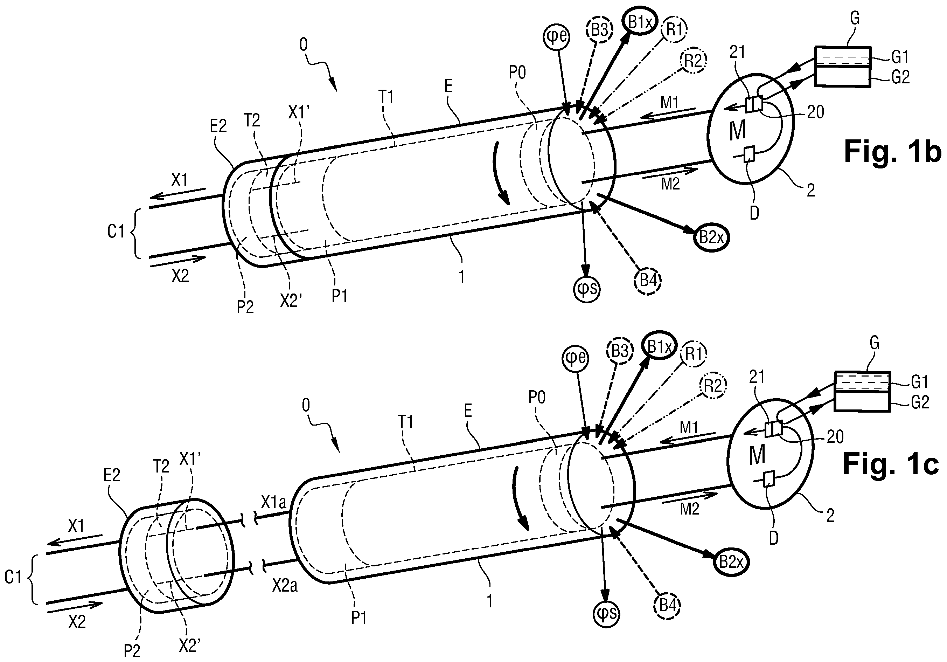

- La figure 1b illustre un système identique à celui de la figue 1a, la seule différence résidant dans le fait que le dispositif d'interface 1 est ici réparti dans deux enceintes E et E2 assemblées mécaniquement entre elles, de manière détachable, ce mode de réalisation permet d'avoir un dispositif d'interface monobloc et rigide lorsque les enceintes sont assemblées entre elles tout en permettant d'interrompre la liaison mécanique entre les tubes X1, X2 et les ports M1, M2 de la machine à hémodialyse en séparant le dispositif d'interface en deux parties respectivement contenues dans les enceintes distinctes E et E2 ;

- La figure 1c illustre un système identique à celui de la figue 1b, la seule différence résidant dans le fait que le dispositif d'interface 1 est ici réparti dans deux enceintes E et E2 qui sont reliées entre elles par un tube veineux intermédiaire X1a et un tube artériel intermédiaire X2a qui sont déformables, les enceintes E, E2 étant ici éloignée l'une de l'autre, ce mode de réalisation permet d'avoir un dispositif d'interface en deux blocs rigides et séparés l'un de l'autre par des tubes souples X1a, X2a dont la longueur peut varier en fonction du besoin, ce mode permet d'interrompre la liaison mécanique entre les tubes X1, X2 et les ports M1, M2 de la machine à hémodialyse en détachant les tubes intermédiaires vis-à-vis de l'une des enceintes E ou E2, les liaisons fluidiques respectives entre les ports M1, M2 et les tubes veineux et artériel X1, X2 pouvant être interrompues soit en actionnant un premier tiroir du dispositif d'interface placé dans la première enceinte E soit en actionnant un second tiroir du dispositif d'interface placé dans la deuxième enceinte E2, on a donc une fonction d'interruption de liaison fluidique qui est redondante et répartie dans des enceintes séparées ;

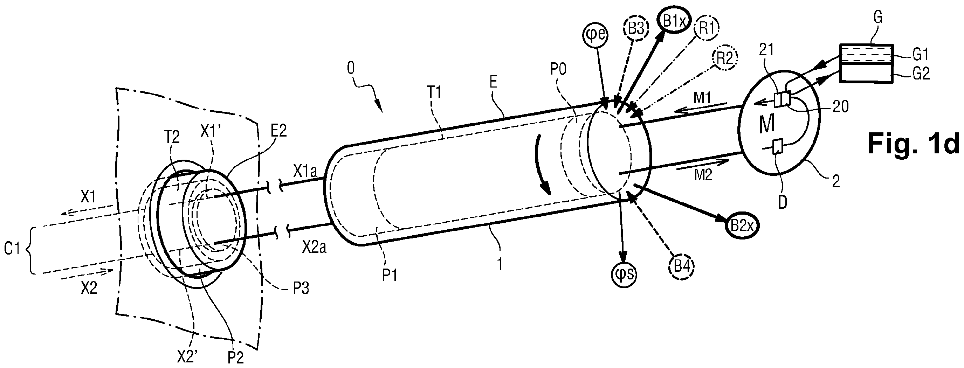

- La figure 1d illustre un système identique à celui de la figue 1c, la seule différence résidant dans le fait que la seconde enceinte E2 est intégrée dans un tissu du patient, elle peut par exemple être implantée en sous cutané ou être implantée de manière transcutanée comme sur cette figure 1d, cette seconde enceinte E2 peut éventuellement présenter une surface externe ostéo intégrée ;

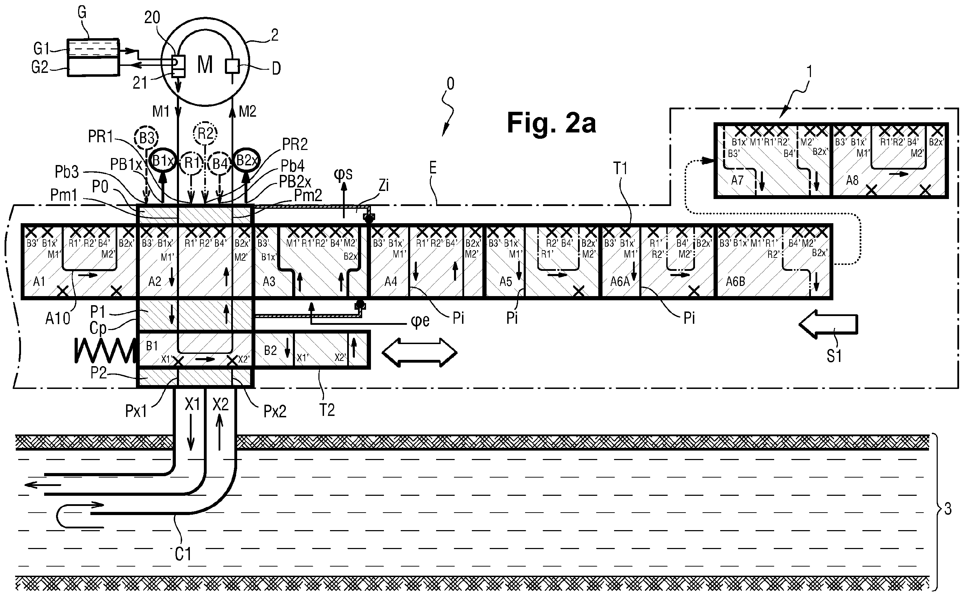

- La figure 2a présente un mode de réalisation du système 0 avec un dispositif d'interface 1 selon l'invention intégré dans une seule enceinte comme sur la figure la ;

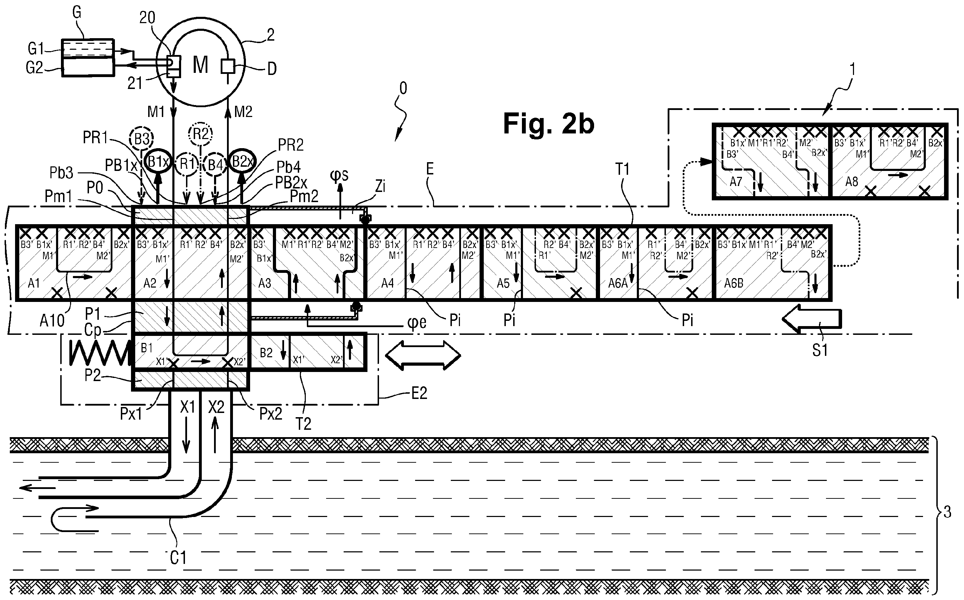

- La figure 2b présente un mode de réalisation du système 0 avec un dispositif d'interface 1 selon l'invention réparti dans deux enceintes E, E2 distinctes et assemblées mécaniquement l'une contre l'autre de manière détachable comme sur la figure 1b ;

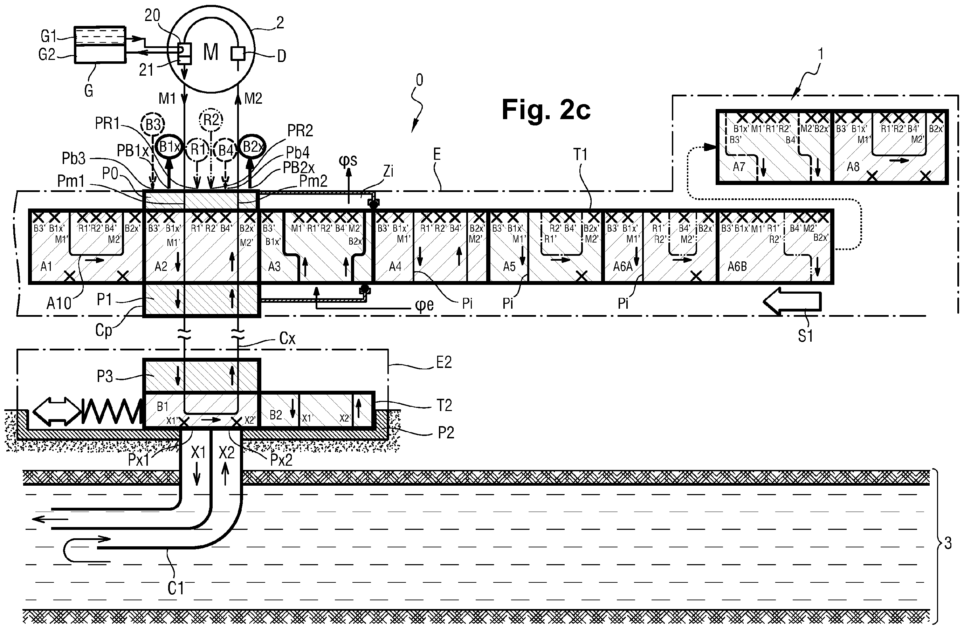

- La figure 2c présente un mode de réalisation du système 0 avec un dispositif d'interface 1 selon l'invention réparti dans deux enceintes E, E2 distinctes reliées entre elles via des tubes intermédiaires souples X1a, X2a comme sur la figure 1c ;

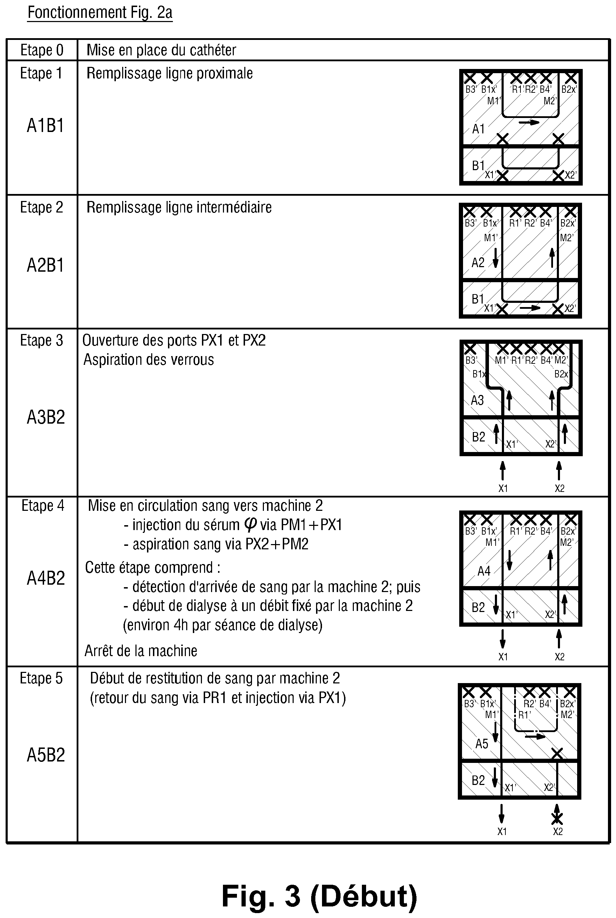

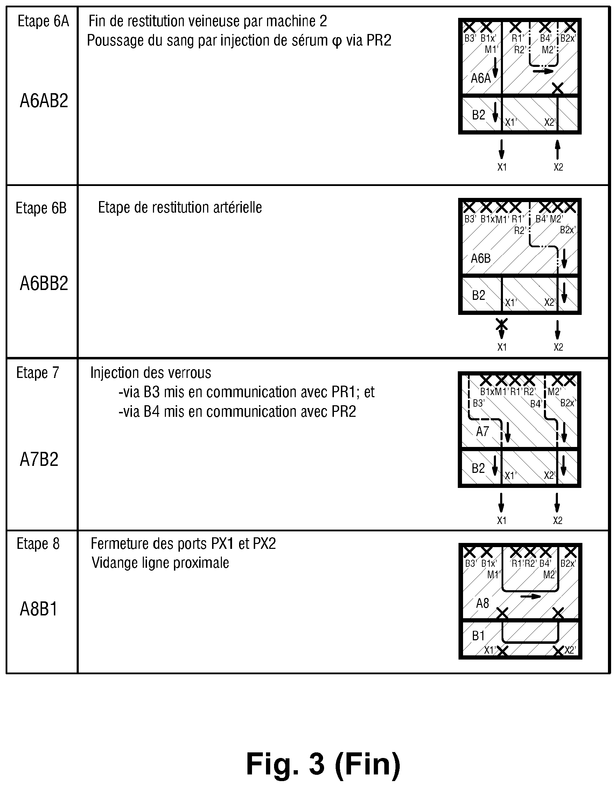

- la figure 3 décrit une succession d'étapes avec de configurations prises par le dispositif d'interface selon l'invention

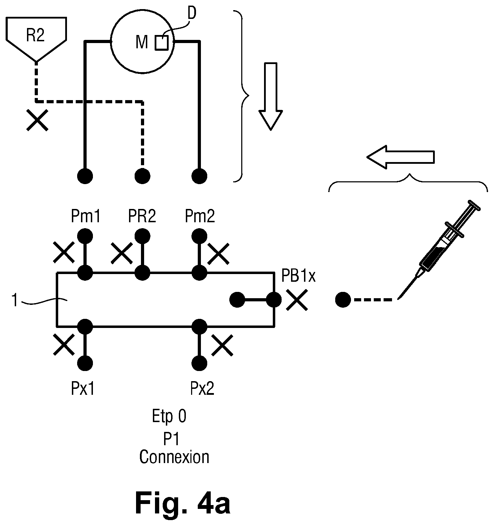

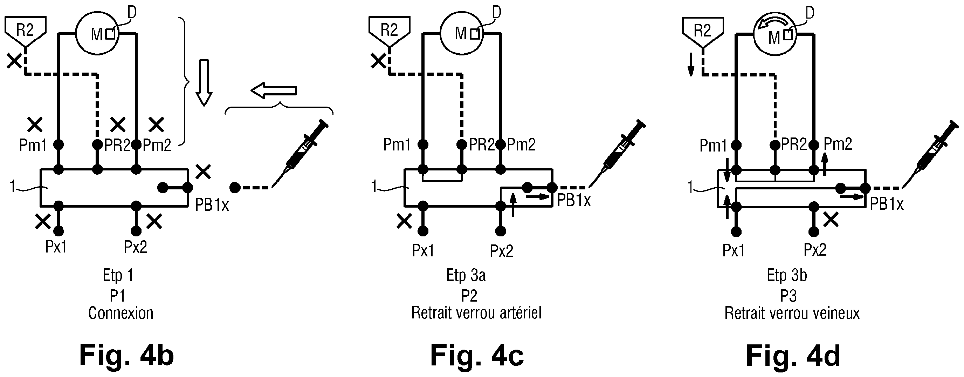

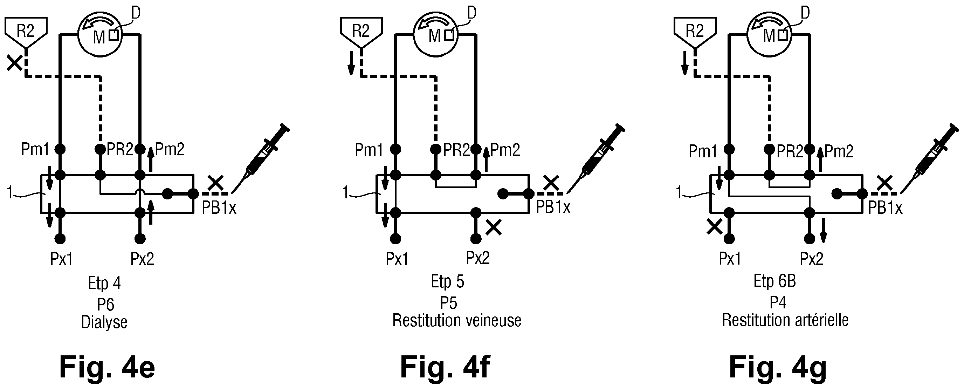

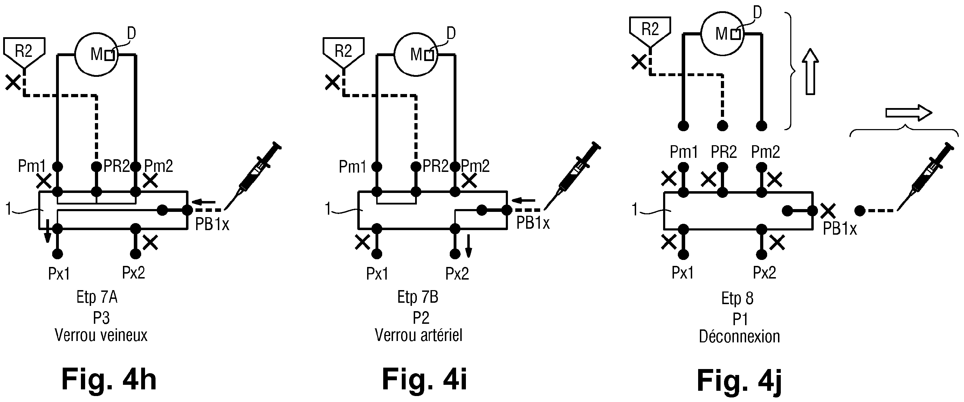

- les figures 4a, 4b, 4c, 4d, 4e, 4f, 4g, 4h, 4i, 4j illustrent respectivement les différentes étapes de fonctionnement Etp0, Etp1, Etp2, Etp3a, Etp3b, Etp4, Etp5, Etp6B, Etp7A, Etp7b, Etp8 d'un dispositif d'interface 1 selon l'invention (ce dispositif d'interface 1 des figures 4a à 4j est plus facile à fabriquer car il présente un nombre réduit de ports et de positions / configurations par rapport à celui des figures 2a à 2c).

### DESCRIPTION DETAILLEE DE L'INVENTION

Selon un aspect général, l'invention porte sur un système d'hémodialyse 0 illustré au travers des figures la à 2c et au travers des figures 4a à 4j. Ce système 0 comprend une machine à hémodialyse 2, un dispositif d'interface 1 et des tubes veineux et artériel X1, X2. Ces tubes X1, X2 sont chacun agencés pour être mis en communication fluidique avec un système circulatoire 3 d'un patient. Ces tubes veineux X1 et artériel X2 appartiennent préférentiellement à un même cathéter C1.

Chacun des tubes X1, X2 est préférentiellement formé dans un conduit souple, par exemple thermoplastique compatible avec un usage médical (polyuréthane, PEEK, silicone ou autre), pour faciliter sa manipulation et son cheminement entre le système circulatoire 3 et le dispositif d'interface 1.

Le tube veineux X1 est essentiellement destiné à l'injection de fluide de la machine 2 vers le patient, et le tube artériel X2 est essentiellement destiné au prélèvement (aspiration) de fluide du patient vers la machine à hémodialyse 2.

La mise en relation des tubes avec le système circulatoire 3 se fait soit via une fistule artérioveineuse, via un cathéter central tunnélisé dans le patient (un cathéter central tunnelisé est un cathéter restant en place dans le corps du patient entre deux hémodialyses) ou via une implantation d'un cathéter d'hémodialyse non tunnélisé (ces tubes pouvant appartenir à un ou plusieurs cathéters). Plus précisément, le dispositif d'interface 1 selon l'invention forme :
- une interface entre la machine à hémodialyse 2 et un tube artériel X2 destiné à être relié audit système circulatoire 3 du patient pour transférer du sang du système circulatoire 3 vers la machine à hémodialyse 2 ; et
- une interface entre la machine à hémodialyse 2 et au moins un tube veineux X1 pour transférer du sang de la machine à hémodialyse vers le système circulatoire 3.

Ce dispositif d'interface 1 comprend :
- un premier port Pm1 adapté à être relié, préférentiellement de manière amovible via un premier raccord, à un port de sortie M1 de la machine à hémodialyse 2 ; et
- un deuxième port Pm2 adapté à être relié, préférentiellement de manière amovible via un second raccord éventuellement solidarisé audit premier raccord, à un port d'entrée M2 de la machine à hémodialyse ;
- un port veineux Px1 pour injecter du sang vers le tube veineux X1, ce port veineux Px1 étant préférentiellement attaché de manière amovible, via un raccord, au tube veineux X1 ; et
- un port artériel Px2 pour recevoir (faire circuler, prélever par exemple par aspiration) du sang du patient provenant du tube artériel X2, ce port artériel Px2 est préférentiellement attaché de manière amovible, via un raccord, au tube artériel X2.

Le deuxième port Pm2 du dispositif d'interface 1 est adapté à transférer du sang du dispositif d'interface 1 vers un port d'entrée M2 de la machine à hémodialyse 2.

La machine à hémodialyse comporte une pompe M agencée pour pomper / faire circuler des fluides de son port d'entrée M2 vers son port de sortie M1.

Le premier port Pm1 du dispositif d'interface 1 est adapté à recevoir du sang provenant du port de sortie M1 pour le transférer vers le tube veineux X1.

La machine à hémodialyse 2 est aussi adaptée à réaliser des échanges entre le fluide qu'elle transfert, en l'occurrence du sang du patient, et un dialysat liquide pour permettre une épuration du fluide (sang). Pour cela la machine à hémodialyse comporte un circuit interne relié d'un côté au port d'entrée M2 et d'un autre côté au port de sortie M1.

La pompe M de la machine à hémodialyse est préférentiellement une pompe péristaltique, pour transférer du fluide du port d'entrée M2 vers le port de sortie M1 avec un débit précisément contrôlé.

Ce circuit interne de la machine à hémodialyse comporte préférentiellement au moins une membrane semi-perméable 20 permettant de réaliser des échanges entre le fluide / le sang et le dialysat formulé chimiquement et/ou des filtres pour épurer le fluide / sang.

Préférentiellement, cette membrane semi-perméable 20 permet des échanges entre un circuit où passe le sang du patient et un circuit de dialysat. Le circuit de dialysat s'étend depuis une réserve de dialysat G1 préalablement formulé vers une réserve de dialysat usagé G2 en passant par une zone de contact contre la membrane semi-perméable 20 pour réaliser les échanges avec le sang du patient.

Le débit et la qualité de ce dialysat peuvent être contrôlés à l'aide d'une pompe à dialysat et/ou de valves et/ou de capteurs d'analyse du dialysat et/ou de capteurs d'analyse de fonctionnement de la membrane semi-perméable 20 et/ou de capteurs de pression de dialysat et/ou de capteur de quantité de dialysat restant dans la réserve G1 et/ou de capteur de quantité de dialysat usagé présent dans la réserve G2.

La machine à hémodialyse 2 comporte aussi un débulleur D destiné à retirer des bulles de gaz contenues dans le fluide transféré via la machine à hémodialyse 2. Ici, le débulleur D est relié en série entre les ports d'entrée M2 et de sortie M1 et il se trouve préférentiellement entre le port M2 et la membrane. Si besoin, la machine peut comporter d'autres débulleurs pour s'assurer de ne pas diffuser de liquide contenant des bulles vers le patient.

La machine à hémodialyse peut aussi comporter un dispositif de détection 21 pour détecter des bulles et/ou des impuretés et/ou un débit de fluide entre ses ports d'entrée M2 et de sortie M1 et/ou une pression du fluide transitant via la machine 2 et/ou un niveau de dialysat dans une réserve de dialysat reliée à la machine 2 pour amener ce dialysat au contact de la membrane semi-perméable.

Ce dispositif de détection 21 est relié à une unité électronique de commande de la machine à hémodialyse 2 pour commander le fonctionnement de la pompe M en fonction de mesures réalisées par ce dispositif de détection 21.

Cette unité électronique peut aussi être reliée à certains au moins desdits capteurs du circuit de dialysat et à des actionneurs du circuit de dialysat pour par exemple de commander des débits de dialysat dans le circuit de dialysat, des dosages de dialysat avec d'autres constituants.

Cette unité électronique peut aussi être reliée à un ou plusieurs capteurs d'état de la membrane semi-perméable pour commander les paramètres de fonctionnement des différents actionneurs dont la pompe M en fonction de mesures réalisées à l'aide de ce/ces capteur(s) d'état.

La machine à hémodialyse peut aussi comporter une interface de communication adaptée à détecter une configuration courante du dispositif d'interface 1 selon l'invention de manière à ajuster le fonctionnement de la machine à hémodialyse en fonction de la configuration courante du dispositif d'interface 1 ainsi détectée. Cette interface de communication peut comprendre une connectique reliant, de manière détachable, la machine à hémodialyse 1 au dispositif d'interface 1.

Cette interface de communication peut être adaptée pour transmettre :
- du dispositif d'interface 1 vers la machine à hémodialyse 2 un signal de configuration courante du dispositif d'interface 1 représentatif d'une configuration courante adoptée par ce dispositif d'interface 1 ; et/ou
- de la machine à hémodialyse 2 vers le dispositif d'interface 1, un signal de changement de configuration, le dispositif d'interface 1 comportant un actionneur, par exemple un moteur, commandant le changement de configuration du dispositif d'interface 1 en fonction du signal de changement de configuration reçu par le dispositif d'interface 1 de manière à faire passer le dispositif d'interface d'une configuration courante vers une autre configuration choisie dans une succession de configurations prédéfinies. Les différentes configurations sélectivement adoptées par le dispositif d'interface seront présentées ci-après.

La figure 3 illustre une succession de configurations que peut adopter sélectivement le dispositif d'interface (par sélectivement, on entend que le dispositif d'interface ne peut adopter à un instant donné qu'une seule configuration parmi les configurations listées).

Pour illustrer ces différentes configurations, on a représenté deux tiroirs T1, T2, chacun de ces tiroirs T1, T2 est mobile par coulissement vis-à-vis deux corps qui lui correspondent et entre lesquels il coulisse à étanchéité. Ici, le dispositif d'interface comporte plusieurs tiroirs et plusieurs corps P0, P1, P2 et éventuellement P3.

Le premier tiroir T1 coulisse à étanchéité entre un corps de base P0 et un corps intermédiaire P1. Ce corps de base P0 porte lesdits premier port Pm1 et deuxième port Pm2 ainsi que d'autre ports du dispositif d'interface qui seront présentés ci-après sous les noms de troisième port Pb1x, quatrième port Pb2x, cinquième port Pb3, sixième port Pb4, port de début de restitution Pr1, port de restitution Pr2.

Le second tiroir T2 coulisse à étanchéité entre deux corps dont un est un corps patient P2 portant les ports veineux et artériel Px1, Px2 et dont l'autre corps est soit ledit corps intermédiaire P1, soit autre corps intermédiaire P3 (dans le cas où le dispositif est réparti dans au moins deux enceintes E, E2).

Dans les applications où le dispositif d'interface 1 est contenu dans une seule enceinte E, comme sur la figure 2a, ou dans deux enceintes E, E2 adjacentes et assemblées rigidement entre elles, comme sur la figure 2b, on préfère utiliser un seul corps intermédiaire P1 contre lequel viennent coulisser à étanchéité les deux tiroirs T1, T2.

Ce corps intermédiaire P1 est traversé de conduits pour permettre le passage de fluide entre des circuits internes Pi au premier tiroir T1, et des circuits internes au second tiroir T2.

Dans d'autres modes de réalisations, comme celui de la figure 2c où l'on souhaite que le dispositif d'interface 1 comporte deux parties mobiles l'une par rapport à l'autre, le dispositif d'interface 1 comprend alors deux enceintes E, E2. La première enceinte E contient le corps de base P0, un corps intermédiaire P1 et le premier tiroir T1 alors que la deuxième enceinte E2 contient un autre corps intermédiaire P3, le corps patient P2 et le second tiroir T2.

Le premier tiroir T1 se trouve dans la première enceinte E où il est monté coulissant à étanchéité entre le corps de base P0 et le premier corps intermédiaire P1, alors que le second tiroir T2 se trouve dans la deuxième enceinte E2 où il est monté coulissant à étanchéité entre le corps patient P2 et le second corps intermédiaire P3, les premier et second corps intermédiaires P1, P3 étant ici reliés fluidiquement entre eux par des conduites souples Cx. On peut faire en sorte que chaque conduite souple Cx ait une extrémité reliée, éventuellement de manière amovible, au premier corps intermédiaire P1 et une seconde extrémité reliée, éventuellement de manière amovible, au second corps intermédiaire P3.

Dans chacun de ces modes des figures 2a à 2c, les tiroirs T1, T2 et les corps P0, P1, P2, P3 sont conformés de manière qu'en fonction des positions respectives des tiroirs T1, T2 par rapport aux corps, on ait des autorisations et/ou des interdictions de passage de fluide entre des ports Pb1x, Pb2x, Pb3, Pb4, Pr1, Pr2, Px1, Px2 portés par les corps P1, P2 conformément à des configurations données du dispositif 1.

Pour cela chaque tiroir T1, T2 comporte plusieurs cellules, en l'occurrence les cellules A1, A2, A3, A4, A5, A6A, A6B, A7, A8 pour le premier tiroir T1 et les cellules B1, B2 pour le second tiroir T2. Le nombre et les configurations de ces cellules peuvent varier en fonction des configurations que l'on souhaite mettre en œuvre.

Chaque configuration donnée du dispositif, est ainsi définie par une position donnée de coulissement du premier tiroir T1 et une position donnée de coulissement du second tiroir T2.

Comme on le comprend de la figures 3, le dispositif d'interface est adapté à sélectivement adopter une première configuration A1B1 (étape 1) et deuxième configuration A4B2 (étape 4).

Dans sa première configuration A1B1 :
le passage de sang entre le premier port Pm1 et le port veineux Px1 est interdit ; et
le passage de sang entre le deuxième port Pm2 et le port artériel Px2 est interdit.

Cette première configuration est utile au moins pour isoler la machine 2 vis-à-vis du système circulatoire du patient.

Dans sa deuxième configuration A4B2 (étape 4), le premier port Pm1 est relié au port veineux Px1 pour permettre le passage de sang du premier port Pm1 vers le port veineux Px1, le deuxième port Pm2 et le port artériel Px2 sont aussi reliés entre eux pour permettre le passage de sang du port artériel Px2 vers le deuxième port Pm2. Dans cette deuxième configuration, seuls les ports Pm1, Px1, Pm2 Px2 sont ouverts et les autres ports du dispositif sont fermés.

Cette deuxième configuration A4B2 est utile pour faire circuler le sang en boucle en passant successivement par le système circulatoire 3, le tube artériel X2, le port artériel Px2, le deuxième port Pm2, le port d'entrée M2, la machine à hémodialyse 2 et son circuit interne, le port de sortie M1, le deuxième port Pm1, le port veineux Px1, le tube veineux X1 et finalement le système circulatoire 3.

Un des avantages du dispositif d'interface 1 selon l'invention est qu'il permet de passer d'une configuration à un autre sans avoir à débrancher manuellement un port de la machine. On peut ainsi autoriser ou interrompre le passage de fluide entre les tubes X1, X2 et la machine à hémodialyse tout en limitant les risques de contamination du patient.

Préférentiellement, le dispositif d'interface est agencé pour que lorsqu'il se trouve dans sa première configuration A1B1, le premier port Pm1 et le deuxième port Pm2 soient alors mis en communication, via un circuit interne A10 au dispositif d'interface 1.

Préférentiellement, le dispositif d'interface est agencé pour que lorsqu'il se trouve dans sa deuxième configuration A4B2, la communication entre le premier port Pm1 et le deuxième port Pm2 via ce circuit interne A10 soit alors interdite.

Ainsi, dans la première configuration, on peut mettre en communication les premier et second ports Pm1 et Pm2 du dispositif 1 via un circuit interne A10 ce qui permet de mettre en relation le port de sortie M1 et le port d'entrée M2 tout en isolant ces ports M1, M2 des tubes artériel et veineux X2, X1.

Cette première configuration permet ainsi d'isoler la machine à hémodialyse vis-à-vis du système circulatoire 3 du patient tout en autorisant la circulation du fluide liquide au travers de la machine à hémodialyse. Des gaz se trouvant dans le circuit de la machine à hémodialyse peuvent être évacués et remplacés par du liquide transitant via le circuit interne A10 du dispositif d'interface. Cette opération de remplissage en liquide de la machine à hémodialyse 2, du dispositif d'interface 1 et des circuits qui s'étendent entre la machine à hémodialyse 2 et le dispositif d'interface 1 est réalisée sans avoir à débrancher les ports d'entrée et de sortie M1, M2.

Comme illustré sur différentes figures la à 2c, le dispositif 1 comporte également un troisième port Pb1x et ce dispositif d'interface 2 est adapté à sélectivement adopter une configuration de déverrouillage A3B2 distincte desdites première et deuxième configurations A1B1, A4B2.

Dans cette configuration de déverrouillage A3B2 Etp3a, le troisième port Pb1x est relié à un seul desdits port veineux Px1 ou port artériel Px2 alors que le premier port Pm1 et le deuxième port Pm2 sont respectivement isolés vis-à-vis du port veineux Px1 et du port artériel Px2, ces ports veineux et artériels étant également isolés l'un de l'autre. Par ports isolés les uns des autres, isolés l'un de l'autre, on entend que ces ports ne communiquent pas fluidiquement entre eux.

Cette configuration de déverrouillage A3B2 permet l'aspiration d'un fluide de verrouillage présent dans le tube veineux X1 et/ou le tube artériel X2 pour pouvoir libérer ce(s) tube(s) et y autoriser le passage de sang / la circulation de fluide liquide. Un fluide de verrouillage est une substance ayant une fonction anticoagulante et une fonction de tampon pour éviter le passage de sang dans le tube qui le contient. Eventuellement, le fluide de verrouillage peut avoir une fonction antiseptique.

Typiquement, après avoir réalisé une hémodialyse, du fluide de verrouillage est injecté dans chaque tube devant rester dans le patient jusqu'à l'hémodialyse suivante. Ce fluide de verrouillage permet d'éviter un bouchage du tube et le besoin de le remplacer.

Grâce au dispositif d'interface 1, on peut injecter ou aspirer du fluide de verrouillage vers le tube veineux X1 et/ou le tube artériel X2 tout en laissant ce(s) tube(s) connecté(s) au dispositif d'interface 1. Encore une fois, on réduit ainsi le besoin de manipulation des raccords et ports et les risques associés pour le patient.

Afin d'aspirer du fluide de verrouillage provenant du tube veineux X1, le système selon l'invention peut aussi comporter une seringue d'aspiration veineuse B1x de fluide de verrouillage reliée au troisième port Pb1x.

Dans le mode de réalisation présenté aux figures 4a à 4j, on constate que le troisième port Px1 est prévu pour être connecté soit au port veineux Px1, soit au port artériel Px2 soit éventuellement à un port de restitution Pr2 destiné à injecter un fluide à restituer au patient (par exemple un sérum physiologique ou médicament liquide). Ainsi, ce même troisième port Px1 est utilisable avec chaque port veineux Px1 et artériel Px2 pour réaliser l'insertion d'un verrou ou son aspiration. Pour cela, ce troisième port est prévu pour être relié à tout de rôle avec une seringue d'aspiration ou avec une seringue d'injection de fluide de verrouillage. Le couplage de chaque seringue est ici effectué manuellement par le praticien d'hémodialyse.

Cette solution est aussi avantageuse puisqu'elle permet de réaliser des opérations de verrouillage des ports veineux ou artériel à l'aide de ce seul troisième port Pb1x.

Pour réaliser un verrouillage veineux ou artériel, il suffit de disposer le dispositif d'interface en configuration de déverrouillage et d'injecter un verrou artériel ou veineux via celui des ports Px1 ou Px2 relié au troisième port Pb1x.

Alternativement, comme illustré sur les figures 2a à 2c, le dispositif d'interface 1 peut aussi comporter un quatrième port Pb2x, ce dispositif d'interface étant en outre agencée pour que lorsqu'il se trouve dans sa configuration de déverrouillage A3B2, le quatrième port Pb2x soit alors relié à l'un desdits ports veineux Px1 ou port artériel Px2 qui n'est pas relié audit troisième port Pb1x, ce quatrième port Pb2x étant isolé vis-à-vis des autres ports du dispositif 1.

Préférentiellement, dans cette configuration de déverrouillage A3B2, les troisième et quatrième ports Pb1x, Pb2x sont isolés l'un de l'autre pour permettre d'aspirer du fluide de verrouillage dans le tube veineux X1 via le troisième port Pb1x et du fluide de verrouillage dans le tube artériel X2 via le quatrième port Pb2x. Cette aspiration peut se faire à l'aide d'un premier moyen d'aspiration relié au seul troisième port Pb1x et d'un second moyen d'aspiration relié au seul quatrième port Pb2x.

A cette fin, le système selon l'invention peut comporter une seringue d'aspiration artérielle B2x de fluide de verrouillage reliée au quatrième port Pb2x pour pouvoir aspirer du fluide de verrouillage provenant du tube artériel X2.

On a ainsi des circuits d'aspiration / moyens d'aspiration distincts l'un de l'autre ce qui limite le risque qu'une opération d'aspiration de fluide de verrouillage dans le tube veineux ou le tube artériel n'interfère avec une opération d'aspiration de fluide de verrouillage dans l'autre de ces tubes.

Selon un mode de réalisation préférentiel, le dispositif d'interface 1 comporte également un cinquième port Pb3, le dispositif d'interface étant adapté pour sélectivement adopter une configuration de verrouillage A7B2 (étape7) distincte desdites première, deuxième configurations et de ladite configuration de déverrouillage A1B1, A4B2, A3B2. Dans cette configuration de verrouillage A7B2, le cinquième port Pb3 est relié audit port veineux Px1 alors que le premier port Pm1, le deuxième port Pm2, le troisième port Pb1x, le port veineux Px1 et le port artériel Px2 sont isolés les uns des autres.

Dans cette configuration de verrouillage A7B2, le quatrième port Pb2x et le cinquième port Pb3 sont également isolés l'un de l'autre.

Dans cette configuration de verrouillage, ce cinquième port Pb3 est relié au port veineux Px1 tout en étant isolé de tous les autres ports du dispositif.

Cette configuration de verrouillage permet l'injection, via le cinquième port Pb3, d'un fluide de verrouillage jusque dans le tube veineux X1 pour pouvoir interdire la présence de sang dans ce tube veineux X1.

Ceci permet de limiter le risque de colmatage de ce tube veineux X1 par du sang coagulé entre deux hémodialyses successives.

A cette fin, le système selon l'invention peut comporter une seringue d'injection veineuse B3 de fluide de verrouillage reliée au cinquième port Pb3 pour pouvoir injecter du fluide de verrouillage vers le tube veineux X1.

Selon un mode de réalisation préférentiel de l'invention, le dispositif d'interface 1 comporte également un sixième port Pb4, le dispositif d'interface 1 étant également adapté pour dans sa configuration de verrouillage A7B2, le sixième port Pb4 soit relié audit port artériel Px2 tout en étant isolé vis-à-vis de tous les autres ports du dispositif.

Le dispositif d'interface selon l'invention permet ainsi de réaliser les opérations de retrait ou de mise en place de fluide de verrouillage dans le tube veineux X1 et le tube artériel X2 tout en laissant la machine à hémodialyse 2 et les tubes veineux et artériel X1, X2 connectés / reliés aux ports concernés du dispositif d'interface. Encore une fois, on limite le risque d'avoir une contamination du système circulatoire par des branchements ou débranchements de ports.

A cette fin, le système selon l'invention peut comporter une seringue d'injection artérielle B4 de fluide de verrouillage reliée au sixième port Pb4 pour pouvoir injecter du fluide de verrouillage vers le tube artériel X2.

Selon un mode de réalisation préférentiel, le dispositif d'interface comporte également un port de restitution Pr2 et le dispositif d'interface 1 est en outre adapté pour sélectivement adopter une configuration de restitution veineuse A6aB2 distincte autres configurations pouvant être adoptées par le dispositif A1B1, A4B2, A3B2, A7B2.

Dans cette configuration de restitution veineuse A6aB2, le port de restitution Pr2 est relié audit deuxième port Pm2 pour pouvoir injecter un fluide de restitution vers la machine à hémodialyse 2, le premier port Pm1 étant alors relié audit port veineux Px1 et isolé de tous les autres ports du dispositif d'interface, et le port artériel Px2 étant au moins isolé vis-à-vis du port veineux Px1, du premier port Pm1 et du deuxième port Pm2.

Dans cette configuration de restitution veineuse A6aB2, le dispositif permet de faire passer un fluide à restituer (fluide de restitution) vers le seul deuxième port Pm2 de la machine à hémodialyse tout en permettant à cette machine à hémodialyse d'injecter du fluide vers le tube veineux X1 via le port de sortie M1 mis en communication avec le premier port Pm1 et le port veineux Px1.

A cette fin, le système selon l'invention peut comporter une réserve R2 de fluide à restituer (par exemple une réserve de sérum physiologique, un dialysat, un médicament sous forme liquide) reliée au port de restitution Pr2 pour pouvoir injecter du fluide à restituer vers le dispositif d'interface lorsque le dispositif d'interface se trouve dans sa configuration de restitution veineuse A6aB2, Etp5.

Comme la machine à hémodialyse 2 forme un circuit s'étendant entre son port d'entrée M2 et son port de sortie M1, la machine à hémodialyse pompe le fluide de restitution de son port d'entrée M2 vers son port de sortie M1 pour ainsi pousser le sang contenu dans la machine et le restituer vers le tube veineux X1 puis vers le système circulatoire 3 du patient.

Ainsi, le volume de sang contenu dans la machine à hémodialyse 2, dans le tube veineux X1 et dans les conduites reliant la machine à hémodialyse 2 au dispositif d'interface 1 peut être restitué au patient. Ceci est particulièrement important pour limiter la quantité de sang perdue par le patient durant l'hémodialyse.

Selon un mode de réalisation préférentiel, le dispositif 1 est en outre adapté pour sélectivement adopter une configuration de restitution artérielle A6bB2, Etp6B distincte des autres configurations que peut adopter le dispositif d'interface A1B1, A4B2, A3B2, A7B2, Etp1, Etp4, Etp5.

Dans cette configuration de restitution artérielle A6bB2 (voir figure 2a à 2c et figure 3), le port de restitution Pr2 peut être relié audit port artériel Px2 pour pouvoir injecter un fluide de restitution vers le tube artériel X2, les autres ports du dispositif étant alors isolés les uns des autres.

Alternativement, le dispositif d'interface peut être adapté pour que dans sa configuration de restitution artérielle Etp6B (voir figure 4g), le port de restitution Pr2 soit relié au deuxième port Pm2 de la machine à hémodialyse alors que le premier port Pm1 de la machine est relié audit port artériel Px2.

Dans cette configuration de restitution artérielle A6bB2, Etp6B, soit le fluide de restitution transite directement du port de restitution Pr2 vers le port artériel sans passer / repasser par la machine, soit le fluide de restitution transite du port de restitution Pr2 vers le deuxième port Pm2, puis ce fluide de restitution transite via la machine M et son dé-bulleur D pour en ressortir et transiter du premier port Pm1 vers le port artériel Px2 (le dispositif d'interface 1 en configuration de restitution artérielle relient ses port Pm1 et Px2 entre eux en les isolant des autres ports du dispositif d'interface).

Dans un mode de réalisation non illustré, il est aussi possible qu'en configuration de restitution les deux ports veineux et artériel soient reliés au port de restitution Pr2 ou éventuellement au premier port Pm1 pour réaliser simultanément la restitution. Cette solution permet un gain de temps lors de la restitution mais elle présente l'inconvénient de ne pas contrôler le volume de fluide restitué à chaque tube X1, X2.

Comme indiqué précédemment, ce fluide de restitution peut être du sérum physiologique.

Dans cette configuration de restitution artérielle A6bB2, le dispositif permet de faire passer un fluide à restituer du port de restitution Pr2 vers le port artériel Px2 tout en isolant les autres ports les uns des autres, le tube artériel X2 peut ainsi être rempli de fluide à restituer et le sang présent dans ce tube est poussé vers le système circulatoire 3.

Cette restitution de sang limite la perte de sang lors de l'hémodialyse et évite le risque de colmatage du tube artériel X2.

Dans un mode de réalisation particulier, le dispositif d'interface peut être adapté pour que lorsqu'il se trouve dans sa première configuration Etp1, son port de restitution Pr2 soit alors relié à l'un au moins desdits du premier port Pm1 et du deuxième port Pm2.

Ainsi, les premier et deuxième ports Pm1, Pm2 peuvent être dégazés en injectant un fluide (liquide) via le port de restitution Pr2.

Préférentiellement, le dispositif d'interface 1 comporte également un mécanisme de commande agencé pour être déplacé dans un premier sens S1 par rapport à l'enceinte E du dispositif d'interface 1. Ce déplacement permet de commander un changement de configuration du dispositif d'interface et par exemples son passage de sa première configuration A1B1 vers sa deuxième configuration deuxième configuration A4B2.

En d'autres termes, le mécanisme de commande permet de commander le passage d'une configuration courante dans laquelle se trouve le dispositif vers une autre configuration du dispositif sélectionnée, cette autre configuration étant sélectionnée parmi les différentes configurations que peut sélectivement adopter le dispositif.

L'enchainement des différentes configurations nécessaires à la réalisation d'une hémodialyse complète sera détaillé plus loin. Idéalement, le mécanisme de commande se déplace uniquement dans le sens S1 de manière à ce que le dispositif ne puisse passer qu'une seule fois dans une configuration donnée.

Préférentiellement, le dispositif d'interface comporte un port d'alimentation en liquide physiologique ϕe (un liquide physiologique est par exemple du sérum physiologique, du dialysat ou de l'eau pure) vers une zone interne Zi du dispositif d'interface et un port d'évacuation de liquide physiologique (ϕs) hors de cette zone interne du dispositif d'interface (1).

Le dispositif d'interface comporte une pluralité de portions de circuits internes Pi au dispositif d'interface et chacune de ces portions de circuits internes étant disposée pour être sélectivement soit reliée entre le port d'alimentation en liquide physiologique ϕe et le port d'évacuation de liquide physiologique ϕs, soit relié à au moins un desdits ports veineux ou artériel Px1, Px2.

Chacune des portions de circuits internes Pi peut ainsi être remplie de liquide physiologique avant d'être reliée avec l'un des ports veineux Px1 ou artériel Px2.

On limite ainsi le risque d'avoir une migration de gaz d'une portion Pi vers le système circulatoire 3.

Grâce à cette caractéristique, on peut faire en sorte que toute portion des circuits internes Pi destinée à être connectée / relier à l'un au moins des ports veineux ou artériel Px1, Px2 soient préalablement remplie de liquide physiologique en la connectant entre le port d'alimentation en liquide physiologique ϕe et le port d'évacuation de liquide physiologique ϕs.

Le port d'alimentation ϕe peut être connecté avec un premier réservoir de liquide physiologique éventuellement équipé d'un débulleur et le port d'évacuation ϕs peut être connecté avec un second réservoir de récupération de liquide physiologique. Ces premier et second réservoirs sont préférentiellement externes au dispositif d'interface.

Alternativement, les ports d'alimentation ϕe et d'évacuation ϕs peuvent être reliés entre eux via une boucle de circulation externe au dispositif d'interface, cette boucle comportant une pompe de circulation, un débulleur et un réservoir de liquide physiologique.

Dans chacun de ces modes, l'objectif est d'alimenter le dispositif d'interface en liquide physiologique ne contenant pas de bulles.

Idéalement, tout au long du passage du dispositif d'interface d'une de ses configurations vers une autre de ses configurations, l'ensemble des portions de circuits internes Pi sont obturées à leurs extrémités en venant frotter contre des surfaces complémentaires internes au dispositif d'interface. On évite ainsi la mise en communication de ports du dispositif pendant un changement de configurations.

Pour réaliser cette obturation on peut, comme sur l'exemple de la figure 2a, faire en sorte que les extrémités des portions de circuits internes Pi destinés à être reliés à l'un des ports veineux ou artériel viennent frotter à étanchéité contre des surfaces complémentaires d'un corps Cp tout au long du passage du dispositif d'interface entre ses première et seconde configurations données.

Dans le mode de réalisation présenté sur la figure 2a, les portions de circuits internes Pi destinés à être reliées à l'un des ports veineux ou artériel sont formées dans des cellules / sections A2, A3, A4, A5, A6a, A6b, A7 d'un tiroir T1. Après remplissage d'une portion de circuit interne Pi en liquide physiologique, cette portion est déplacée par le mécanisme de commande vers le corps Cp qui l'obture jusqu'à ce qu'elle arrive dans une position prédéterminée par rapport aux ports du dispositif qu'elle doit mettre en communication fluidique.

Au moment de la mise en communication fluidique entre une portion de circuit interne Pi et un port veineux ou artériel, le liquide physiologique qui se trouve dans cette portion de circuit interne Pi passe vers le port auquel il est relié sans risque d'introduction de gaz / d'air.

Le fonctionnement du dispositif va maintenant être explicité en référence à la figure 3.

Par convention, chacune des cellules A1 à A8 et B1, B2 des tiroirs T1, T2 présentent plusieurs références chacune formée d'une lettre d'un chiffre suivi du caractère « ' ». Chacune de ces références dans la cellule correspond au port identifié par un « P » et suivi de la même lettre et du même chiffre.

Lorsqu'une référence avec le caractère « ' » se trouve en face d'un « X », cela signifie que cette cellule est agencée pour interdire le passage de fluide vers le port de référence correspondante.

Par exemple, sur la cellule A1 du tiroir T1, les références B3', B1x', R1',R2', B4' et B2x' sont chacune en face de « X » ce qui implique que lorsque cette cellule A1 est dans sa position d'utilisation, alors l'ensemble des ports correspondants Pb3, Pb1x, Pr1, Pr2, Pb4 et Pb2x sont obturés.

A contrario, lorsque qu'une référence d'une cellule se trouve le long d'un trait dessiné dans cette cellule alors cela signifie qu'un circuit interne est prévu pour relier entre eux deux des ports du dispositif lorsque cette cellule est en position d'utilisation.

Les ports reliés par ce circuit interne sont ceux désignés par les références situées le long de ce trait.

Ainsi, sur la cellule A1, on voit le long du trait les références M1', M2' ce qui indique que lorsque cette cellule A1 se trouve dans sa position d'utilisation alors les ports Pm1 et Pm2 du dispositif sont mis en relation fluidique l'un avec l'autre.

Autre exemple, lorsque les cellules A2 et B1 sont dans leurs positions d'utilisation respectives (configuration A2B1) alors on voit un trait s'étendant au travers de chacune des cellules A2 et B1 avec les références M1' et M2' placées le long de ce trait. Cela signifie que lorsque ces cellules A2 et B1 sont dans leurs positions d'utilisation alors les ports Pm1 et Pm2 sont reliés entre eux via ce circuit interne qui passe par les cellules A2, B1.

Une première étape d'utilisation du système 0 consiste à relier mécaniquement les ports du dispositif d'interface 1 à la machine à hémodialyse 2, aux tubes X1, X2 et à certains au moins des différents appareils B1x, B2x, B3, B4, R1, R2 à utiliser et à placer le dispositif 1 dans sa première configuration (étape 1).

L'appareil R1 peut être un port spécifique de la machine à hémodialyse 2 utilisé uniquement lors de la restitution de sang en fin d'hémodialyse ou éventuellement une poche de sang ou de médicament devant être injectée en fin d'hémodialyse.

Dans cette première configuration A1B1 (étape 1) la machine à hémodialyse 2 est actionnée pour faire circuler du liquide entre les ports Pm1 et Pm2.

Le liquide circule alors en boucle entre la machine 2 et le dispositif d'interface 1 en passant par le débulleur D qui permet d'évacuer les bulles du liquide.

La machine 2 réalise cette circulation jusqu'à ce qu'elle détecte, ou qu'un opérateur détecte, que le circuit de la machine et le dispositif d'interface ne contient que du liquide.

Dans un mode de réalisation comme celui de la figure 2c ou 1c où les premier et second tiroirs T1, T2 sont très éloignés l'un de l'autre, on peut prévoir des moyens pour remplir en liquide le circuit reliant ces tiroirs. A cette fin, les premier et second tiroirs présentent des cellules respectives A2 et B1 qui, lorsqu'elles sont en position d'utilisation, permettent une circulation de fluide entre les ports Pm1 et Pm2 via un circuit traversant ces cellules A2, B1 (voir l'exemple de la figure 2c et l'étape 2 de la figure 3 / configuration A2B1).

Après remplissage en liquide du dispositif d'interface 1 et du circuit interne de la machine à hémodialyse 2, le mécanisme de commande (par exemple un moteur ou un électroaimant) est actionné pour faire passer le dispositif d'interface dans sa configuration de déverrouillage A3B2 (étape 3) qui autorise l'actionnement des seringues d'aspiration veineuse et artérielle B1x et B2x. Le fluide de verrouillage contenu dans les tubes X1, X2 est alors aspiré. Eventuellement, l'actionnement des seringues peut être motorisé et commandé par l'unité de commande de la machine à hémodialyse.

Une fois les tubes X1, X2 libérés du fluide de verrouillage, le mécanisme de commande est commandé pour que le dispositif d'interface passe dans sa deuxième configuration A4B2 (étape 4). Dans cette configuration le tube artériel X2 est mis en communication avec le port d'entrée M2 et le tube veineux X1 est mis en communication avec le port de sortie M1. Le sang du patient peut alors circuler en boucle via la machine à hémodialyse 2 pour y être dialysé.

Une fois l'hémodialyse effectuée, et de manière optionnelle, on peut vouloir restituer un liquide particulier au patient. Dans ce cas, ce liquide peut être amené via un port de début de restitution PR1 qui appartient au dispositif d'interface. Ce port Pr1 peut être relié à une sortie de sang R1 spécifique de la machine à hémodialyse ou à une poche R1 de sang ou de médicament.

A cette fin, le mécanisme de commande est actionné pour que le dispositif d'interface passe en configuration de début de restitution A5B2 (étape 5). Dans cette configuration le port PR1 est mis en communication avec le port Pm2 et le tube veineux X1 est mis en communication avec le port de sortie M1. Le liquide particulier à restituer passe alors successivement via le port Pr1, le port Pm2, le port d'entrée M2, la machine à hémodialyse, le port de sortie M1, le port Pm1 et enfin il arrive au tube veineux X1.

Il est à noter que cette étape peut être optionnelle et que le système selon l'invention peut ne pas comporter d'appareil R1, de port PR1, et ne jamais passer par la configuration A5B2. Dans ce cas la cellule A4 pourrait être supprimée du tiroir T1.

Une fois cette étape 5 terminée ou à défaut d'étape 5, une fois l'étape 4 terminée, le mécanisme de commande est commandé pour que le dispositif d'interface 1 passe en configuration de restitution veineuse A6AB2 (étape 6A) qui autorise :
- la fermeture du port artériel Px2 / du tube artériel X2 ; et
- la mise en relation de l'appareil périphérique R2 avec le port d'entrée machine M2 via le port de restitution Pr2 et le deuxième port Pm2.

Cet appareil périphérique R2 peut être une réserve R2 de fluide à restituer lorsque le dispositif d'interface se trouve dans sa configuration de restitution veineuse A6aB2.

Typiquement, le fluide de la réserve R2 est du sérum physiologique utilisé pour pousser le sang restant dans la machine vers le tube veineux X1.

Le dispositif de détection 21 peut détecter l'arrivée du sérum physiologique et ainsi détecter la fin de l'opération de restitution veineuse.

Une fois cette étape 6A terminée, le mécanisme de commande est actionné pour que le dispositif d'interface 1 passe en configuration de restitution artérielle A6BB2 (étape 6B) ce qui autorise la fermeture du port veineux Px1 et du premier port Pm1 et la mise en relation de l'appareil périphérique R2 avec le tube artériel X2 via le port de restitution Pr2 et le port artériel Px2.

Le fluide de restitution contenu dans la réserve R2 peut alors circuler au travers du dispositif d'interface 1 pour chasser le sang contenu dans le tube artériel X2 vers le patient.

L'opération de restitution est terminée lorsqu'un volume de liquide prédéterminé a été restitué vers le tube artériel X2.

Une fois cette étape 6B terminée, le mécanisme de commande est actionné pour que le dispositif d'interface 1 passe en configuration de verrouillage A7B2 (étape 7) qui d'une part autorise la mise en communication de l'appareil B3 avec le tube veineux X1 via le port Pb3 et le port Px1 et d'autre part autorise la mise en communication de l'appareil B4 avec le tube artériel X2 via le port Pb4 et le port Px2.

Dans cette configuration de verrouillage tous les autres ports du dispositif sont fermés.

L'appareil B3 est une seringue d'injection veineuse B3 de fluide de verrouillage dédiée à l'injection vers le port X1 et l'appareil B4 est une seringue d'injection artérielle B4 de fluide de verrouillage dédiée à l'injection vers le port X2.

Après avoir réalisé cette injection des verrous, on peut soit directement débrancher le dispositif d'interface vis-à-vis du patient et vis-à-vis de la machine à hémodialyse ou alors commander la fermeture de l'ensemble des ports du dispositif d'interface pour pouvoir le débrancher, ou alors commander la vidange de la ligne proximale qui relie le dispositif d'interface à la machine à hémodialyse.

C'est cette dernière étape de vidange qui est représentée à la figure 8, le dispositif étant alors en configuration vidange A8B1. Dans cette configuration, A8B1, les ports Px1 et Px2 sont isolés et les ports Pm1 et Pm2 mis en communication pour que le fluide contenu entre le dispositif d'interface 1 et la machine 2 puisse être retiré.

Nous allons maintenant présenter le cas où la présente invention est utilisée pour réaliser des hémodialyses sur des patients équipés en permanence de tubes X1 et X2.

Dans ce cas, en référence au mode de la figure 1d, il est possible de prévoir que le dispositif 1 comporte un pilier ostéointégré présentant un connecteur implanté sur le patient où aboutissent chacun des tubes X1 et X2.

Le port veineux Px1 du dispositif d'interface 2 est relié au tube veineux X1 via un tube veineux intermédiaire X1a dont une première extrémité est reliée au port veineux Px1 et dont une seconde extrémité aboutie dans un second connecteur amovible du patient.

Le port artériel Px2 du dispositif d'interface 2 est relié au tube artériel X2 via un tube artériel intermédiaire X2a dont une première extrémité est reliée au port artériel Px2 et dont une seconde extrémité aboutie dans le second connecteur amovible du patient.

Le connecteur implanté sur le patient et le second connecteur amovible du patient sont agencés pour pouvoir être assemblés l'un avec l'autre de manière réversible et de manière que lorsqu'ils sont assemblés l'un avec l'autre alors le tube veineux X1 est relié de manière étanche avec le tube veineux intermédiaire X1a et le tube artériel X2 est relié de manière étanche avec le tube artériel intermédiaire X2a.

Le connecteur implanté sur le patient comporte des valves disposées pour autoriser le passage de fluide entre les tubes implantés X1, X2 et tubes intermédiaire X1a, X2a lorsque le connecteur implanté est assemblé avec second connecteur amovible et qu'une commande des valves mobile entre une configuration de fermeture des valves et une configuration d'ouverture des valves se trouve dans sa configuration d'ouverture des valves.

Il est à noter que la commande des valves peut être déplacée entre sa configuration de fermeture des valves et sa configuration d'ouverture des valves tant que le connecteur implanté est assemblé avec le second connecteur amovible. La commande des valves est préférentiellement prévue pour qu'en cas de désassemblage du second connecteur amovible vis-à-vis du connecteur implanté, la commande des valves se déplace automatiquement de sa configuration d'ouverture des valves vers sa configuration de fermeture des valves. On évite ainsi que les tubes implantés X1, X2 débouchent directement à l'extérieur du patient.

En conclusion, le dispositif d'interface 1 selon l'invention et le système d'hémodialyse 0 qui l'intègre permettent dans leurs différents modes de réalisations de :
- réaliser plusieurs fonctions nécessaires à l'hémodialyse du patient ;
- réduire le risque d'infection du patient durant l'hémodialyse en limitant les opérations manuelles de branchement ou débranchement des tubes veineux et artériel vis à vis des ports veineux et artériel Px1, Px2 et des ports de sortie et entrée machine M1, M2 vis-à-vis des premier et deuxième ports Pm1, Pm2 du dispositif d'interface (toutes les opérations d'isolement fluidique ou de mise en relation avec des appareils nécessaires à l'hémodialyse sont réalisables sans avoir à débrancher un seul port du dispositif vis-à-vis d'un de ces appareils) ;
- limiter le risque d'erreurs de manipulation lors de la procédure de la séance d'hémodialyse 0 ;
- limiter le risque de contamination par réduction du besoin de manipulation de ports ;
- réduire le temps d'intervention du personnel soignant pendant l'hémodialyse ;
- réduire le temps de préparation et de fin de séance d'hémodialyse (ceci permet un gain de temps d'environ 10% de la durée totale d'une séance d'hémodialyse pour le patient et pour le personnel soignant) .

Ce dispositif d'interface 1 est en outre compatible avec différents types machine à hémodialyse 2 sans nécessiter de les modifier.

## Revendications

1. Dispositif d'interface (1) entre une machine à hémodialyse (2) et au moins un tube veineux (X1) destiné à être relié au système circulatoire (3) d'un patient pour transférer du sang de la machine à hémodialyse vers le système circulatoire, ce dispositif d'interface (1) comprenant :
- un premier port (Pm1) adapté à être relié à un port de sortie (M1) de la machine à hémodialyse (2);
- un port veineux (Px1) pour injecter du sang vers le tube veineux (X1) ;
le dispositif d'interface étant adapté à sélectivement adopter une première configuration (A1B1; Etp1) dans laquelle le passage de sang entre le premier port (Pm1) et le port veineux (Px1) est interdit et une deuxième configuration (A4B2; Etp4) dans laquelle le premier port (Pm1) est relié au port veineux (Px1) pour permettre le passage de sang du premier port (Pm1) vers le port veineux (Px1), le dispositif d'interface (1) étant également adapté à former une interface entre la machine à hémodialyse (2) et un tube artériel (X2) destiné à être relié audit système circulatoire (3) du patient pour transférer du sang du système circulatoire vers la machine à hémodialyse, ce dispositif d'interface (1) comprenant également :
- un deuxième port (Pm2) adapté à être relié à un port d'entrée (M2) de la machine à hémodialyse ;
- un port artériel (Px2) pour recevoir du sang du patient provenant du tube artériel (X2), le dispositif d'interface étant en outre adapté pour interdire le passage de sang entre le deuxième port (Pm2) et le port artériel (Px2) lorsque le dispositif se trouve dans sa première configuration (A1B1 ; Etp1) et pour autoriser le passage de sang entre le deuxième port (Pm2) et le port artériel (Px2) lorsque le dispositif se trouve dans sa deuxième configuration (A4B2 ; Etp4), **caractérisé en ce que** le dispositif d'interface comporte également un troisième port (Pblx), le dispositif d'interface étant adapté pour sélectivement adopter une configuration de déverrouillage (A3B2 ; Etp3a) distincte desdites première et deuxième configurations (A1B1, A4B2 ; Etp1 ; Etp4), dans cette configuration de déverrouillage (A3B2) le troisième port (Pblx) étant relié à un seul desdits port veineux (Px1) ou port artériel (Px2) alors que le premier port (Pm1) et le deuxième port (Pm2) sont respectivement isolés vis-à-vis du port veineux (Px1) et du port artériel (Px2), ces ports veineux et artériel étant également isolés l'un de l'autre.

2. Dispositif d'interface (1) selon la revendication 1, en outre adapté pour mettre en communication, via un circuit interne (A10) au dispositif d'interface, le premier port (Pm1) et le deuxième port (Pm2) lorsque ce dispositif d'interface se trouve dans sa première configuration (A1B1) et pour interdire la communication entre le premier port (Pm1) et le deuxième port (Pm2) via ce circuit interne (A10) lorsque ce dispositif d'interface se trouve dans sa deuxième configuration (A4B2 ; Etp4).

3. Dispositif d'interface selon l'une quelconque des revendications 1 ou 2, comportant également un quatrième port (Pb2x), le dispositif d'interface étant tel que lorsqu'il se trouve dans sa configuration de déverrouillage (A3B2), le quatrième port (Pb2x) est relié à l'un desdits port veineux (Px1) ou port artériel (Px2) qui n'est pas relié audit troisième port (Pblx) et isolé vis-à-vis des autres ports du dispositif.

4. Dispositif d'interface selon l'une quelconque des revendications 1 à 3, comportant également un cinquième port (Pb3), le dispositif d'interface étant adapté pour sélectivement adopter une configuration de verrouillage (A7B2) distincte desdites première, deuxième configurations et le la configuration de déverrouillage (A1B1, A4B2, A3B2), dans cette configuration de verrouillage (A7B2), le cinquième port (Pb3) est relié audit port veineux (Px1) alors que le premier port (Pm1), le deuxième port (Pm2), le troisième port (Pblx), le port veineux (Px1) et le port artériel (Px2) sont isolés les uns des autres.

5. Dispositif d'interface selon la revendication 4, comportant également un sixième port (Pb4), le dispositif d'interface étant adapté pour que dans sa configuration de verrouillage (A7B2), le sixième port (Pb4) soit relié audit port artériel (Px2) tout en étant isolé vis-à-vis de tous les autres ports du dispositif d'interface (1).

6. Dispositif d'interface selon l'une quelconque des revendications 1 à 5, comportant également un port de restitution (Pr2), le dispositif d'interface étant adapté pour sélectivement adopter une configuration de restitution veineuse (A6aB2, Etp5) distincte des autres configurations du dispositif (A1B1, A4B2, A3B2, A7B2, Etp1 ,Etp4), dans cette configuration de restitution veineuse (A6aB2, Etp5), le port de restitution (Pr2) est relié audit deuxième port (Pm2) pour pouvoir injecter un fluide de restitution vers la machine à hémodialyse (2), le premier port (Pm1) étant alors relié audit port veineux (Px1) et isolé de tous les autres ports du dispositif d'interface, et le port artériel (Px2) étant au moins isolé vis-à-vis du port veineux (Px1), du premier port (Pm1) et du deuxième port (Pm2).

7. Dispositif d'interface selon la revendication 6, adapté pour sélectivement adopter une configuration de restitution artérielle (A6bB2, Etp6B) distincte des autres configurations que peut adopter le dispositif d'interface (A1B1, A4B2, A3B2, A7B2, Etp5), dans cette configuration de restitution artérielle (A6bB2, Etp6B), le port de restitution (Pr2) est relié audit port artériel (Px2) pour pouvoir injecter un fluide de restitution vers le tube artériel (X2), les autres ports du dispositif étant alors isolés les uns des autres.

8. Dispositif d'interface selon l'une quelconque des revendications 6 ou 7, dans lequel le dispositif d'interface est adapté pour que lorsqu'il se trouve dans sa première configuration (Etpl), son port de restitution (Pr2) est alors relié à l'un au moins desdits du premier port (Pm1) et du deuxième port (Pm2).

9. Dispositif d'interface selon l'une quelconque des revendications 6 à 8, dans lequel le dispositif d'interface comporte un mécanisme de commande agencé pour être déplacé dans un premier sens (S1) par rapport à une enceinte (E) du dispositif d'interface (1), ce déplacement commandant un passage du dispositif de sa première configuration (A1B1) vers sa deuxième configuration deuxième configuration (A4B2).

10. Dispositif d'interface selon l'une quelconque des revendications 1 à 9, dans lequel le dispositif d'interface comporte un port d'alimentation en liquide physiologique (ϕe) vers une zone interne (Zi) du dispositif d'interface et un port d'évacuation de liquide physiologique (ϕs) hors de cette zone interne du dispositif d'interface (1), le dispositif d'interface comportant une pluralité de portions de circuits internes (Pi) au dispositif d'interface, chacune de ces portions de circuits internes étant disposée pour être sélectivement soit reliée entre le port d'alimentation en liquide physiologique (ϕe) et le port d'évacuation de liquide physiologique (ϕs), soit relié à au moins un desdits ports veineux ou artériel (Px1, Px2).

11. Système d'hémodialyse (0) comprenant un dispositif d'interface (1) selon l'une quelconque des revendications 1 à 10 et une machine à hémodialyse (2), le premier port (Pm1) du dispositif d'interface (1) étant relié de manière amovible au port de sortie (M1) de la machine à hémodialyse (2) et le deuxième port (Pm2) du dispositif d'interface (1) étant relié de manière amovible au port d'entrée (M2) de la machine à hémodialyse, la machine à hémodialyse comportant une pompe (M) agencée pour faire circuler des fluides de son port d'entrée (M2) vers son port de sortie (Ml), le système d'hémodialyse comprenant également un tube veineux (X1) et un tube artériel (X2), le tube veineux (X1) étant reliée au port veineux (Px1) du dispositif d'interface (1), ce tube veineux (X1) étant destiné à être relié au système circulatoire (3) d'un patient pour transférer, via le dispositif d'interface (1), du sang de la machine à hémodialyse (2) vers le système circulatoire (3), le tube artériel (X2) étant relié au port artériel (X2) du dispositif d'interface (1), ce tube artériel (X2) étant destiné à être relié audit système circulatoire (3) du patient pour transférer, via le dispositif d'interface (1), du sang du système circulatoire (3) vers la machine à hémodialyse (2) .

12. Système d'hémodialyse selon la revendication 11, dans lequel le système comporte une seringue d'aspiration veineuse (B1x) de fluide de verrouillage reliée au troisième port (Pblx) pour pouvoir aspirer du fluide de verrouillage provenant du tube veineux (X1).

13. Système d'hémodialyse selon la revendication 11 combinée à la revendication 3, dans lequel le système comporte une seringue d'aspiration artérielle (B2x) de fluide de verrouillage reliée au quatrième port (Pb2x) pour pouvoir aspirer du fluide de verrouillage provenant du tube artériel (X2).

## Patentansprüche

1. Schnittstellenvorrichtung (1) zwischen einer Hämodialysemaschine (2) und mindestens einem Venenschlauch (X1), der dazu bestimmt ist, mit dem Kreislaufsystem (3) eines Patienten verbunden zu werden, um Blut von der Hämodialysemaschine zum Kreislaufsystem zu übertragen, wobei diese Schnittstellenvorrichtung (1) umfasst:
- einen ersten Port (Pm1), der angepasst ist, um mit einem Auslassport (M1) der Hämodialysemaschine (2) verbunden zu werden;
- einen Venenport (Px1) zum Injizieren von Blut in den Venenschlauch (X1);
wobei die Schnittstellenvorrichtung angepasst ist, um selektiv eine erste Konfiguration (A1B1; Etp1), in der der Blutdurchlass zwischen dem ersten Port (Pm1) und dem Venenport (Px1) untersagt ist, und eine zweite Konfiguration (A4B2; Etp4) anzunehmen, in der der erste Port (Pm1) mit dem Venenport (Px1) verbunden ist, um den Blutdurchlass von dem ersten Port (Pm1) zum Venenport (Px1) zu gestatten, wobei die Schnittstellenvorrichtung (1) ferner angepasst ist, um eine Schnittstelle zwischen der Hämodialysemaschine (2) und einem Arterienschlauch (X2) zu bilden, der dazu bestimmt ist, mit dem genannten Kreislaufsystem (3) des Patienten verbunden zu werden, um Blut vom Kreislaufsystem zur Hämodialysemaschine zu übertragen, wobei diese Schnittstellenvorrichtung (1) ferner umfasst:
- einen zweiten Port (Pm2), der angepasst ist, um mit einem Einlassport (M2) der Hämodialysemaschine verbunden zu werden;
- einen Arterienport (Px2) zum Empfangen von Blut des Patienten, das aus dem Arterienschlauch (X2) stammt, wobei die Schnittstellenvorrichtung ferner angepasst ist, um den Blutdurchlass zwischen dem zweiten Port (Pm2) und dem Arterienport (Px2) zu untersagen, wenn sich die Vorrichtung in ihrer ersten Konfiguration (A1B1; Etp1) befindet, und um den Blutdurchlass zwischen dem zweiten Port (Pm2) und dem Arterienport (Px2) zu gestatten, wenn sich die Vorrichtung in ihrer zweiten Konfiguration (A4B2; Etp4) befindet, **dadurch gekennzeichnet, dass** die Schnittstellenvorrichtung ferner einen dritten Port (Pblx) umfasst, wobei die Schnittstellenvorrichtung angepasst ist, um selektiv eine Freigabekonfiguration (A3B2; Etp3a) anzunehmen, die sich von der ersten und zweiten Konfiguration (A1B1, A4B2; Etp1; Etp4) unterscheidet, wobei in dieser Freigabekonfiguration (A3B2) der dritte Port (Pblx) mit nur einem Port aus dem Venenport (Px1) oder dem Arterienport (Px2) verbunden ist, während der erste Port (Pm1) und der zweite Port (Pm2) gegenüber dem Venenport (Px1) bzw. dem Arterienport (Px2) isoliert sind, wobei der Venenport und der Arterienport ferner voneinander isoliert sind.

2. Schnittstellenvorrichtung (1) nach Anspruch 1, die ferner daran angepasst ist, um über einen internen Kreislauf (A10) innerhalb der Schnittstellenvorrichtung den ersten Port (Pm1) und den zweiten Port (Pm2) in Kommunikation zu bringen, wenn sich diese Schnittstellenvorrichtung in ihrer ersten Konfiguration (A1B1) befindet, und um die Kommunikation zwischen dem ersten Port (Pm1) und dem zweiten Port (Pm2) über diesen internen Kreislauf (A10) zu untersagen, wenn sich diese Schnittstellenvorrichtung in ihrer zweiten Konfiguration (A4B2; Etp4) befindet.

3. Schnittstellenvorrichtung nach einem der Ansprüche 1 oder 2, ferner umfassend einen vierten Port (Pb2x), wobei die Schnittstellenvorrichtung derart ist, dass, wenn sie sich in ihrer Freigabekonfiguration (A3B2) befindet, der vierte Port (Pb2x) mit einem Port aus dem Venenport (Px1) oder dem Arterienport (Px2) verbunden ist, der nicht mit dem genannten dritten Port (Pblx) verbunden und gegenüber den anderen Ports der Vorrichtung isoliert ist.

4. Schnittstellenvorrichtung nach einem der Ansprüche 1 bis 3, ferner umfassend einen fünften Port (Pb3), wobei die Schnittstellenvorrichtung angepasst ist, um selektiv eine Sperrkonfiguration (A7B2) anzunehmen, die sich von der ersten Konfiguration, der zweiten Konfiguration und der Freigabekonfiguration (A1B1, A4B2, A3B2) unterscheidet, wobei in dieser Sperrkonfiguration (A7B2) der fünfte Port (Pb3) mit dem Venenport (Px1) verbunden ist, während der erste Port (Pm1), der zweite Port (Pm2), der dritte Port (Pblx), der Venenport (Px1) und der Arterienport (Px2) voneinander isoliert sind.

5. Schnittstellenvorrichtung nach Anspruch 4, ferner umfassend einen sechsten Port (Pb4), wobei die Schnittstellenvorrichtung angepasst ist, damit in ihrer Sperrkonfiguration (A7B2) der sechste Port (Pb4) mit dem Arterienport (Px2) verbunden ist, während er gegenüber allen anderen Ports der Schnittstellenvorrichtung (1) isoliert ist.

6. Schnittstellenvorrichtung nach einem der Ansprüche 1 bis 5, ferner umfassend einen Restitutionsport (Pr2), wobei die Schnittstellenvorrichtung angepasst ist, um selektiv eine Venenrestitutionskonfiguration (A6aB2, Etp5) anzunehmen, die sich von den anderen Konfigurationen der Vorrichtung (A1B1, A4B2, A3B2, A7B2, Etp1, Etp4) unterscheidet, wobei in dieser Venenrestitutionskonfiguration (A6aB2, Etp5) der Restitutionsport (Pr2) mit dem genannten zweiten Port (Pm2) verbunden ist, um ein Restitutionsfluid in Richtung der Hämodialysemaschine (2) injizieren zu können, wobei der erste Port (Pm1) dann mit dem Venenport (Px1) verbunden und von allen anderen Ports der Schnittstellenvorrichtung isoliert ist, und wobei der Arterienport (Px2) mindestens gegenüber dem Venenport (Px1), dem ersten Port (Pm1) und dem zweiten Port (Pm2) isoliert ist.

7. Schnittstellenvorrichtung nach Anspruch 6, die angepasst ist, um selektiv eine Arterienrestitutionskonfiguration (A6bB2, Etp6B) anzunehmen, die sich von den anderen Konfigurationen (A1B1, A4B2, A3B2, A7B2, Etp5), die die Schnittstellenvorrichtung annehmen kann, unterscheidet, wobei in dieser Arterienrestitutionskonfiguration (A6bB2, Etp6B) der Restitutionsport (Pr2) mit dem Arterienport (Px2) verbunden ist, um ein Restitutionsfluid in Richtung des Arterienschlauchs (X2) injizieren zu können, wobei die anderen Ports der Vorrichtung dann voneinander isoliert sind.

8. Schnittstellenvorrichtung nach einem der Ansprüche 6 oder 7, bei der die Schnittstellenvorrichtung angepasst ist, damit, wenn sie sich in ihrer ersten Konfiguration (Etp1) befindet, ihr Restitutionsport (Pr2) dann mit mindestens einem Port aus dem ersten Port (Pm1) und dem zweiten Port (Pm2) verbunden ist.

9. Schnittstellenvorrichtung nach einem der Ansprüche 6 bis 8, bei der die Schnittstellenvorrichtung einen Steuermechanismus umfasst, der ausgebildet ist, um in eine erste Richtung (S1) in Bezug auf eine Umfassung (E) der Schnittstellenvorrichtung (1) bewegt zu werden, wobei diese Bewegung einen Übergang der Vorrichtung von ihrer ersten Konfiguration (A1B1) in ihre zweite Konfiguration (A4B2) steuert.

10. Schnittstellenvorrichtung nach einem der Ansprüche 1 bis 9, bei der die Schnittstellenvorrichtung einen Zuführport zum Zuführen von physiologischer Flüssigkeit (ϕe) zu einer inneren Zone (Zi) der Schnittstellenvorrichtung umfasst, sowie einen Abführport zum Abführen von physiologischer Flüssigkeit (ϕs) aus dieser inneren Zone der Schnittstellenvorrichtung (1), wobei die Schnittstellenvorrichtung eine Vielzahl interner Kreislaufabschnitte (Pi) innerhalb der Schnittstellenvorrichtung umfasst, wobei jeder dieser internen Kreislaufabschnitte angeordnet ist, um selektiv entweder zwischen dem Zuführport zum Zuführen von physiologischer Flüssigkeit (ϕe) und dem Abführport zum Abführen von physiologischer Flüssigkeit (ϕs) oder mit mindestens einem Port aus dem der Venen- oder Arterienport (Px1, Px2) verbunden zu werden.

11. Hämodialysesystem (0), umfassend eine Schnittstellenvorrichtung (1) nach einem der Ansprüche 1 bis 10 und eine Hämodialysemaschine (2), wobei der erste Port (Pm1) der Schnittstellenvorrichtung (1) auf lösbare Weise mit dem Auslassport (M1) der Hämodialysemaschine (2) verbunden ist und der zweite Port (Pm2) der Schnittstellenvorrichtung (1) auf lösbare Weise mit dem Einlassport (M2) der Hämodialysemaschine verbunden ist, wobei die Hämodialysemaschine eine Pumpe (M) umfasst, die ausgebildet ist, um Fluide von ihrem Einlassport (M2) zu ihrem Auslassport (M1) zu leiten, wobei das Hämodialysesystem ferner einen Venenschlauch (X1) und einen Arterienschlauch (X2) umfasst, wobei der Venenschlauch (X1) mit dem Venenport (Px1) der Schnittstellenvorrichtung (1) verbunden ist, wobei dieser Venenschlauch (X1) dazu bestimmt ist, mit dem Kreislaufsystem (3) eines Patienten verbunden zu werden, um über die Schnittstellenvorrichtung (1) Blut von der Hämodialysemaschine (2) zum Kreislaufsystem (3) zu übertragen, wobei der Arterienschlauch (X2) mit dem Arterienport (X2) der Schnittstellenvorrichtung (1) verbunden ist, wobei dieser Arterienschlauch (X2) dazu bestimmt ist, mit dem genannten Kreislaufsystem (3) des Patienten verbunden zu werden, um über die Schnittstellenvorrichtung (1) Blut von dem Kreislaufsystem (3) zur Hämodialysemaschine (2) zu übertragen.

12. Hämodialysesystem nach Anspruch 11, bei dem das System eine Venensaugspritze (B1x) zum Saugen von Sperrfluid umfasst, die mit dem dritten Port (Pblx) verbunden ist, um Sperrfluid, das aus dem Venenschlauch (X1) stammt, absaugen zu können.

13. Hämodialysesystem nach Anspruch 11, kombiniert mit Anspruch 3, bei dem das System eine Arteriensaugspritze (B2x) zum Saugen von Sperrfluid umfasst, die mit dem vierten Port (Pb2x) verbunden ist, um Sperrfluid, das aus dem Arterienschlauch (X2) stammt, absaugen zu können.

## Claims

1. Interface device (1) between a hemodialysis machine (2) and at least one venous tube (X1) intended to be connected to a patient's circulatory system (3) for transferring blood from the hemodialysis machine to the circulatory system, this interface device (1) comprising:
- a first port (Pm1) adapted to be connected to an output port (M1) of the hemodialysis machine (2);
- a venous port (Px1) to inject blood into the venous tube (X1);
the interface device being adapted to selectively adopt a first configuration (A1B1; Etp1) in which the passage of blood between the first port (Pm1) and the venous port (Px1) is prohibited and a second configuration (A4B2; Etp4) in which the first port (Pm1) is connected to the venous port (Px1) to allow blood to pass from the first port (Pm1) to the venous port (Px1), the interface device (1) being also adapted to form an interface between the hemodialysis machine (2) and an arterial tube (X2) for connection to said patient's circulatory system (3) to transfer blood from the circulatory system to the hemodialysis machine, said interface device (1) also comprising:
- a second port (Pm2) adapted to be connected to an input port (M2) of the hemodialysis machine;
- an arterial port (Px2) for receiving blood from the patient via the arterial tube (X2), the interface device being further adapted to prohibit the passage of blood between the second port (Pm2) and the arterial port (Px2) when the device is in its first configuration (A1B1; Etp1) and to allow the passage of blood between the second port (Pm2) and the arterial port (Px2) when the device is in its second configuration (A4B2; Etp4), characterized the interface device comprises a third port (Pblx), the interface device being adapted to selectively adopt an unlocking configuration (A3B2; Etp3a) distinct from said first and second configurations (A1B1, A4B2; Etp1; Etp4), in this unlocking configuration (A3B2) the third port (Pblx) being connected to only one of said venous port (Px1) or arterial port (Px2) while the first port (Pm1) and the second port (Pm2) are respectively isolated from the venous port (Px1) and the arterial port (Px2), these venous and arterial ports also being isolated from each other.

2. Interface device (1) according to claim 1, further adapted to connect, via an internal circuit (A10) to the interface device, the first port (Pm1) and the second port (Pm2) when this interface device is in its first configuration (A1B1) and to prohibit communication between the first port (Pm1) and the second port (Pm2) via this internal circuit (A10) when this interface device is in its second configuration (A4B2; Etp4).

3. Interface device according to any of claims 1 or 2, also comprising a fourth port (Pb2x), the interface device being such that when in its unlocking configuration (A3B2), the fourth port (Pb2x) is connected to one of said venous port (Px1) and arterial port (Px2) which is not connected to said third port (Pblx) and isolated from the other ports of the device.

4. Interface device according to any of claims 1 to 3, also comprising a fifth port (Pb3), the interface device being adapted to selectively adopt a locking configuration (A7B2) distinct from said first, second and unlocking configurations (A1B1, A4B2, A3B2), in this locking configuration (A7B2), the fifth port (Pb3) being connected to said venous port (Px1) while the first port (Pm1), the second port (Pm2), the third port (Pblx), the venous port (Px1) and the arterial port (Px2) are isolated from each other.

5. Interface device according to claim 4, also comprising a sixth port (Pb4), the interface device being adapted so that in its locking configuration (A7B2), the sixth port (Pb4) is connected to said arterial port (Px2) while being isolated from all other ports of the interface device (1).

6. Interface device according to any of claims 1 to 5, also including a release port (Pr2), the interface device being adapted to selectively adopt a venous release configuration (A6aB2, Etp5) distinct from the other configurations of the device (A1B1, A4B2, A3B2, A7B2, Etp1, Etp4), in this venous release configuration (A6aB2, Etp5), the release port (Pr2) is connected to said second port (Pm2) to be able to inject a release fluid into the hemodialysis machine (2), the first port (Pm1) then being connected to said venous port (Px1) and isolated from all other ports of the interface device, and the arterial port (Px2) being at least isolated from the venous port (Px1), from the first port (Pm1) and the second port (Pm2).

7. Interface device according to claim 6, adapted to selectively adopt an arterial restitution configuration (A6bB2, Etp6B) distinct from other configurations that the interface device may adopt (A1B1, A4B2, A3B2, A7B2, Etp5), in this arterial restitution configuration (A6bB2, Etp6B), the restitution port (Pr2) is connected to said arterial port (Px2) to be able to inject a restitution fluid into the arterial tube (X2), the other ports of the device being then isolated from each other.

8. Interface device according to any of claims 6 or 7, wherein said interface device is adapted so that, when in its first configuration (Etp1), its return port (Pr2) is connected to at least one of said first port (Pm1) and second port (Pm2).

9. Interface device according to any of claims 6 to 8, wherein said interface device comprises a control mechanism arranged to be moved in a first direction (S1) with respect to an enclosure (E) of the interface device (1), this movement controlling a passage of the device from its first configuration (A1B1) to its second configuration (A4B2).

10. Interface device according to any of claims 1 to 9, wherein said interface device includes a physiological liquid supply port (ϕe) to an inner area (Zi) of the interface device and a physiological liquid discharge port (cps) outside this inner area of the interface device (1), the interface device comprising a plurality of internal circuit portions (Pi) to the interface device, each of these internal circuit portions being arranged to be selectively connected either between the physiological liquid supply port (ϕe) and the physiological liquid discharge port (ϕs), or connected to at least one of said venous or arterial ports (Px1, Px2).

11. Hemodialysis system (0) comprising an interface device (1) according to any of claims 1 to 10 and a hemodialysis machine (2), the first port (Pm1) of the interface device (1) being detachably connected to the output port (M1) of the hemodialysis machine (2) and the second port (Pm2) of the interface device (1) being detachably connected to the input port (M2) of the hemodialysis machine, the hemodialysis machine having a pump (M) arranged to circulate fluids from its inlet port (M2) to its outlet port (M1), the hemodialysis system also comprising a venous tube (X1) and an arterial tube (X2), the venous tube (X1) being connected to the venous port (Px1) of the interface device (1), this venous tube (X1) being intended to be connected to the circulatory system (3) of a patient to transfer, via the interface device (1), blood from the hemodialysis machine (2) to the circulatory system (3), the arterial tube (X2) being connected to the arterial port (X2) of the interface device (1), this arterial tube (X2) being intended to be connected to said circulatory system (3) of the patient to transfer, via the interface device (1), blood from the circulatory system (3) to the hemodialysis machine (2).

12. The hemodialysis system according to claim 11 in combination with claim 3, wherein the system comprises a venous syringe (B1x) for aspirating locking fluid connected to the third port (Pblx) to be able to draw locking fluid from the venous tube (X1).

13. The hemodialysis system according to claim 12 in combination with claim 3, wherein the system comprises an arterial syringe (B2x) for aspirating locking fluid connected to the fourth port (Pb2x) to be able to draw locking fluid from the arterial tube (X2).
